# EUROPEAN PATENT SPECIFICATION

(11) **EP 1 202 705 B1**
(45) Date of publication and mention of the grant of the patent: **05.12.2007**
(21) Application number: 00951699.8
(22) Date of filing: 04.08.2000
(51) Int. Cl.: A61K 8/25, A61K 6/033, A61L 27/00, A61Q 11/00

(54) **COMPOSITION FOR TREATING DENTIN HYPERSENSITIVITY**
ZUSAMMENSETZUNG ZUR BEHANDLUNG VON DENTIN HYPERSENSITIVITÄT
COMPOSITION DESTINEE A TRAITER LA HYPERSENSITIVITE DE LA DENTINE

(30) Priority: 05.08.1999 US 147438 P; 18.10.1999 US 419514
(43) Date of publication of application: 08.05.2002
(73) Proprietor: BLOCK DRUG COMPANY, INC., West Trenton, New Jersey 08628 (US)
(72) Inventor: DODD, Gregory, P., Hackensack, NJ 07601 (US); HALECKY, Alan, A., West Orange, NJ 07052 (US); MARKOWITZ, Kenneth, J., Fanwood, NJ 07023 (US)
(74) Representative: Reeves, Julie Frances
(86) International application number: PCT/GB2000/003015
(87) International publication number: WO 2001/010392

(56) References cited:
- EP-A- 0 381 961
- WO-A-95/33441
- US-A- 3 929 493
- US-A- 5 074 916
- DATABASE CHEMABS [Online] CHEMICAL ABSTRACTS SERVICE, COLUMBUS, OHIO, US; Database accession no. 1986:613952 XP002157906 & CS 225 193 A (S. KOPRIVOVA ET AL.) 13 February 1984 (1984-02-13)
- DATABASE WPI Derwent Publications Ltd., London, GB; AN 1995196355 XP002157908 & JP 07 112023 A (UNIV MATSUMOTO SHIKA), 2 May 1995 (1995-05-02)
- DATABASE WPI Derwent Publications Ltd., London, GB; AN 1987029654 XP002157909 & AU 58436 86 A (J.J. CHEETHAM), 18 December 1986 (1986-12-18)
- DATABASE CHEMABS [Online] CHEMICAL ABSTRACTS SERVICE, COLUMBUS, OHIO, US; Database accession no. 1991:589840 XP002157907 & JP 03 165773 A (TDK CORP) 17 July 1991 (1991-07-17)
- DATABASE WPI Derwent Publications Ltd., London, GB; AN 1989153849 XP002157910 & JP 01 093439 A (KORU COAT KK), 12 April 1989 (1989-04-12)

## Description

### FIELD OF THE INVENTION

The present invention relates generally to compositions of amorphous bioactive particulates consisting essentially of calcium, silicon and oxygen and methods for their use in treating dentin hypersensitivity.

### BACKGROUND OF THE INVENTION

It is known that products such as bioactive particulates can be used in restoring or repairing defects in calcified tissues such as periodontal pockets, extraction sites, treatments of dentin hypersensitivity, bone tissue formation and the like.

The biocompatability of bioactive particulates in restoring or repairing defects in calcified tissues such as bone tissue formation, as evidenced by tight apposition of hard tissue to implanted glassy particles, was first demonstrated in 1971. Specifically, the use of bioactive glass was originally reported by Hench et al. in J. Biomed. Mater. Res. Symp., 2: 117-141 (1971). Bone grows by apposition onto bioactive glass placed adjacent to bony surfaces and new bone may be conducted subsequently, over short distances.

Granules of bioactive glass have been described in subsequent bone tissue formation applications. U.S. Pat. No. 4,239,113 describes a composition for the preparation of bone cement. U.S. Pat. No. 5,658,332 discloses a method to form osseous tissue in defects in sites in the appendicular skeleton or sites exhibiting reduced metabolic state using bioactive glass granules containing 40 to 58% SiO₂, 10 to 30% NaO, 10 to 30% CaO, and 0 to 10% P₂O₅, and in the size range of from 200 to 300 micrometers.

In the area of repairing or restoring defects in dental tissues, bioactive particulate has been used for periodontal osseous defect repair (U.S. Pat. No. 4,851,046) utilizing a size range of 90-710 µm and a compositional range of 40-55 wt. % SiO₂, 10-30 wt. % CaO, 10-35 wt. % Na₂O, 2-8 wt. % CaF_{2,} and 0-10 wt. % B₂O₃). U.S. Pat. No. 4,851,046 describes compositions containing particles of bioactive glass of various size ranges for use in periodontal applications. U.S. Pat. No. 5,204,106 discloses that particles of bioactive glass within the narrow size range of 280 to 425 micrometers elicit a distinctively altered biologic response from that described previously.

Bioactive particulates have also been used in the treatment of dentin hypersensitivity. Dentin is a hard but resilient calcareous tissue that mechanically supports the dental enamel. The dentin encloses and protects the pulp. The dentin also has a large number of continuous dentinal tubules which connect the pulp cavity with the dentinoenamel/cementum junction. These fine dentinal tubules radiate out from the pulp. The problem of tooth sensitivity arises when the enamel and cementum are worn away from the tooth by either improper oral hygiene practices or as a result of periodontal disease and/or its treatment. The dentinal tubules are fluid-filled exposing the central core of the tooth to the external environment. Hence, when subjected to air current, tactile or thermal stimulation, the pulpal nerves are irritated, leading to the perception of pain. Under a predominant theory called the hydrodynamic theory to explain the reason for hypersensitive dentin, the stimuli conveyed through dentinal tubules irritates nerves distributed in the dental pulp causing pain in these areas. Suppressing sensitive dentin and ameliorating or alleviating the pain might be achieved by sealing the dentinal tubules, thus blocking the conductor of the sensation by physical or chemical means.

In earlier applications to provide for partial occlusion of dentinal tubules, U.S. Patent No. 5,735,942 ("'942 patent") discloses a bioactive melt-derived glass composition for the treatment of tooth hypersensitivity. The '942 patent discloses the following composition by weight percentage: 40-60 SiO₂, 10-30 CaO, 10-35 Na₂O, 2-8 P₂O₅, 0-25 CaF₂ and 0-10 B₂O₃, with particle size of less than about 10 µm with some particles are about 2 µm or less and some larger than 2 µm. The '942 patent notes that 60% SiO₂ is the maximum silica limit for bioactive melt-derived glass.

In a melt-derived process, there are three reactions which lead to the development of a porous hydrated silica-gel layer: ion exchange, silica dissolution, and condensation of silanols to form siloxane-bonded hydrated silica chains or rings. As the SiO₂ content increases in melt-derived glasses, the rates of these reactions decrease, reducing the availability of Ca⁺² ions in solution and the ability to develop the silica-gel layer on the surface. The result is the reduction and eventual elimination of the bioactivity of the melt-derived glasses as the SiO₂ content approaches 60%. The process imposes narrow effective compositional zones which hinder the formulator's ability to modify and tailor the material for a specific application.

Bioactive glasses prepared by the melt-derived process and as taught in prior art ('942 patent) are processed in a platinum crucible at high temperatures, typically about 1350°C or so. Therefore, melt-derived glasses are costly and difficult to transfer to production facilities. The high-temperature processing plus the multiple handling steps render bioactive glass prepared by the melt-derived process rather expensive, effectively limiting the practicality of using bioactive glass for oral care products.

Besides the high production cost, there are other disadvantages inherent with the prior art. First, it is difficult to maintain the very high purity required for optimal bioactivity due to the melt-derived process itself and the low silica and high alkali content of traditional bioactive glass compositions. Secondly, the prior art requires processing steps which involve grinding, polishing, fritting, sieving, etc., all of which expose the bioactive powder to potential contaminants that may negatively effect bioactivity. Third, there is a compositional limitation imposed on bioactive glasses and glass-ceramics made by conventional high-temperature processes due to the extremely high equilibrium liquidus temperature of SiO₂ of 1713°C, and the extremely high viscosity of silicate melts with high SiO₂ content.

In another reference, US Patent No. 5,874,101 (the "'101 patent") proposes a bioactive gel prepared by an improved process, a sol-gel process which includes a drying step for the treatment of tooth hypersensitivity. As with existing bioactive glasses and glass/ceramics and as disclosed in the the '942 patent, the bioactive glasses in the '101 patent are confined to a specific compositional zone in the Na₂O-CaO-P₂O₅-SiO₂ system defined mainly in silica content (Ogin, M., Ohuchi, F and Hench, L.L., Compositional dependence of the formation of calcium phosphate films on bioglass, J. Biomed. Mater. Res. 1980, 14, 55-64 and Ohtsuki, C., Kokubo, T., Takatsuka, K. and Yamamuro, T., Compositional dependence of bioactivity of glasses in the system CaO-SiO2-P2O3: its in vitro evaluation, J. Ceram. Soc. Japn. 1991, 99, 1-6). The '101 patent discloses a composition in weight percent of 40-90 SiO₂, 4-45 CaO, 0-10 Na₂O, 2-16 P₂O₅, 0-25 CaF₂, 0-4 B₂O₃, 0-8 K₂O and 0-5 MgO.

None of the foregoing prior art references provide bioactive particulates which are simple and easy to prepare, and which promote the initiation of calcium-containing mineral to restore and/or repair calcified mineral tissues.

U.S. Pat. No. 3,929,493 describes a dental base material comprised of an organic acid salt of calcium suspended in a network of silica gel.

AU-B-58436/86 describes an opaque dental pit and fissure sealant composition comprising a polymerisable acrylic resin, and hydrophobic precipitated particulate silica filler containing calcium silicate, which functions as both suspending agent and opaquing agent.

WO 95/33441 describes a phosphate-free composition for treating hypersensitive teeth comprising a cationically charged colloid (which may comprise a metal halide, oxide, hydroxide, silicate or acetate).

### SUMMARY OF THE INVENTION

This invention relates to an inorganic or organic / inorganic composite composition which consists essentially of CaO and SiO₂ for treating dentine hypersensitivity. The organic/inorganic composite material is preferably an amorphous binary oxide material of the system CaO-SiO₂ having been prepared by the addition of a soluble calcium source to a suitable silicon-donating precursor, such as sodium silicate.

The invention relates to an inorganic composition which consists essentially of CaO and SiO₂, which, when lodged in the tubules initiates the formation of calcium-containing mineral inside the dentinal tubules for the alleviation of the pain associated with sensitive teeth.

Preferably the composite composition should have a particle size in the size range of about 10 microns or less, advantageously with most in the range of about 2 microns or less.

The bioactive composition of the invention may be produced and/or obtained by the amorphous precipitation of inorganic materials.

### IN THE DRAWINGS

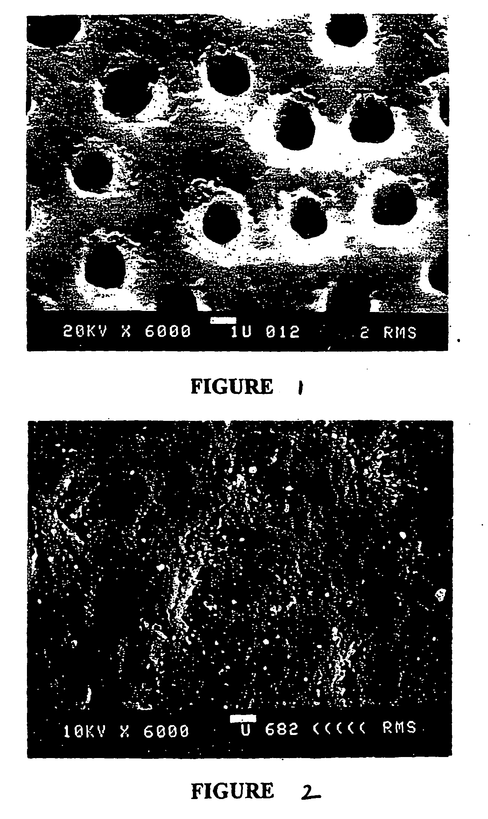

Figure 1 is a scanning electron photomicrograph (20 KV X 6000 magnification) of an untreated dentin disc surface. Figure 2 shows a similarly prepared dentin disc surface after being treated with materials made by the dilute precipitation process.

### DETAINED DESCRIPTION OF THE INVENTION

The inventors have found a surprisingly low cost and convenient-to-manufacture bioactive materials that restore defects in calcified tissues, including the alleviation of pain associated with hypersensitive teeth. The inventors have also found a bioactive glass that is conducive to mineralization by varying the processing parameters and the wet chemistry of the materials.

Bioactive Particulate Composition. Current bioactive materials, including bioactive glass, often rely on a complicated list of oxides, including P₂O₅, Na₂O, B₂O₃, K₂O, MgO, Al₂O₃, TiO₂, Ta₂O₅ and fluoride salts, including CaF₂. We surprisingly found that nucleating crystal growth can be initiated by utilizing only a binary oxide system based essentially exclusively on Si, O and Ca⁺². The bioactive materials prepared in accordance with the present invention, a CaO-SiO₂ containing inorganic composite derived from naturally occurring analogs, are capable of depositing a layer of apatite or other calcium-containing mineral, similar in composition and crystallinity to those mineral phases that predominate in normal dentin once the material's surface is placed in contact with blood, saliva or simulated body fluid.

We have also found that by retaining bioactivity while removing these species from the composition, we can lower the cost of the material, allow greater freedom in processing and eliminate possible organoleptic and toxicological problems. The simplicity of the chemical composition of the present invention, which not only aids in facilitating processing and lowering costs, but also allows flexibility to tailor the composition to the intended application. We found a material tailored to nucleating calcium-containing mineral for the treatment of a specific problem, tooth sensitivity, and removed excipients that may be a necessity for biological activity when used as an osteo-inductive material elsewhere in the body.

Unlike most currently-used bioactive materials that act in part by encouraging in-growth and tissue formation via osteoblasts and other hard tissue cells, the materials of the present invention surprisingly do not require cellular adjuvants. Additionally, the compositions in accordance with the present invention are able to induce the precipitation of apatite and calcium-containing mineral from simulated body fluids without the aid of bone morphogenic proteins, dentin phosphoproteins, odontoblasts or their host cells, or a defined collagen matrix. The ability to induce mineralization without the need for organic adjuvants optimizes this material for use in the oral environment.

In addition, many of these currently-used materials need highly specialized properties such as porosity and mechanical strength suited to their specific clinical use. For example, Li, Clark and Hench showed that there is a minimum rate of hydroxyapatite formation which is necessary to be an effective mineralizing agent and that this rate is a function of both composition and microstructure (Li et. al., 'An Investigation of Bioactive Glass Powders by Sol-Gel Processing, J. of Applied Biomaterials, Vol. 2, 231-239 [1991]). The inventors have surprisingly found that the materials of the present invention do not need the porosity and mechanical strength of the prior art bioglasses to work in dental applications, so that when lodged in the tubules they initiate the formation of calcium-containing mineral inside the dentinal tubules.

The inventors have also surprisingly found that the bioactive materials of the present invention to be bioactive well beyond the compositional boundary for melt-derived glasses of the prior art with a less porous microstructure.

Although it is not necessary, other oxides including P₂O₅, Na₂O, B₂O₃, K₂O, MgO, Al₂O₃, TiO₂, Ta₂O₅ and fluoride salts, including CaF₂, as commonly called for in the compositions in the prior art, can be used in conjunction with the binary oxide system of the present invention.

A preferred and exemplary application for the bioactive materials of the present invention is in the treatment of dentin hypersensitivity. In microscopic examination of exposed sensitive dentin surfaces show that clear differences exist between sensitive and non-sensitive regions of dentin. Sensitive dentin is permeated by open tubules. In contrast, non-sensitive tooth surfaces are characterized by tubules that are sealed off from the external environment by naturally occurring mineral deposits or coverings. We found that once bound to the dentin surface or lodged within the tubule, the amorphous material of the present invention initiates nucleation and mineralization stimulated by the salivary environment, sealing the tubule from the external stimuli by depositing a layer of apatite or other calcium containing mineral, similar to native tooth mineral in composition and crystallinity.

Carriers suitable for use with the composition for use in the treatment of dentin hypersensitivity are preferably hydroxylic materials such as water, polyols and mixtures thereof. Polyols, sometimes referred to as humectants, include glycerol, sorbitol, propylene glycol, xylitol, polypropylene glycol, polyethylene glycol, hydrogenated corn syrup and mixtures thereof. Particularly preferred as the carrier is a liquid mixture of 3 - 30% water, 0- 90% glycerol and 0 - 80% sorbitol. Generally the amount of the carrier will range from about 25 to 99.9% by weight, preferably from about 70 - 95% by weight.

When the compositions of this invention are in the form of a toothpaste or gel for the treatment of dentin hypersensitivity, there will typically be included a natural or synthetic thickening agent in an amount from about 0.1 - 10%, preferably about 0.5 - 5% by weight. Thickeners may include hydroxypropyl methylcellulose, hydroyethyl cellulose, sodium carboxymethylcellulose, xanthan gum, tragacanth gum, karaya gum, gum arabic, Irish moss, starch, alginates and carrageenans.

Surfactants are normally included in compositions for the treatment of dentin hypersensitivity. These surfactants may be of the anionic, nonionic, cationic or amphoteric type. Most preferred are sodium lauryl sulfate, sodium dodecylbenzene sulfonate and sodium laurylsarcosinate. Surfactants are usually present in an amount from about 0.5 - 80% by weight. For anti-caries protection, a source of fluoride ion will normally be present in the oral compositions. Fluoride sources include sodium fluoride, potassium fluoride, stannous monofluorophosphate, calcium fluoride, stannous fluoride and sodium monoflurophosphate. These sources should release from about 25 - 3500 ppm fluoride ion. The anti-caries agents will be present in an amount from about 0.05 - 3.0% by weight, preferably about 0.5 - 1.0%.

Flavors that are usually present in oral care compositions are those based on oils of spearmint and peppermint. Examples of other flavoring agents include menthol, clove, wintergreen, eucalyptus and aniseed. Flavors may range in concentration from about 0.1 - 5.0%. Sweetening agents such as saccharin, sodium cyclamate, aspartame, sucrose and the like may be included at levels from about 0.1 - 5.0% by weight. Other additives may be incorporated including preservatives, silicones, other synthetic or natural polymers, anti-gingivitis actives, anti-tartar agents, whitening agents and other desirable products often found in a conventional toothpaste such as baking soda and peroxide. It is also advantageous that these materials are compatible with conventional desensitizing agents such as potassium nitrate, potassium chloride and potassium bicarbonate, as well as novel desensitizing agents such as those described in U.S. Patent No. 5,589,159.

Numerous vehicles are present for the oral delivery of active agents in the treatment of dentin hypersensitivity, including but not limited to, pastes, gels, rinses, powders, gums, dental floss, slurries and solutions and although each is not described it is within the scope of the present invention.

**Preparation.** It is an advantage of the current invention that the materials are easy to fabricate eliminating the costly and time consuming processing procedures involved with the melt-derived and sol-gel derived bioactive materials in the prior art.

The process of the present invention can be used to prepare the bioactive materials of the present invention, which are based exclusively on Si, O and Ca⁺².

### Precipitation-Based Process Producing Inorganic Particles.

In the most preferred method of the precipitation process, a "dilute precipitation process," an amorphous CaO-SiO₂ inorganic material is produced by the reaction of a dilute silicate source, i.e., aqueous potassium or sodium silicate solutions with a soluble calcium source as previously described. This procedure allows for the manipulation of both the composition of the final precipitate as well as physical characteristics such as the final size or concentration. Materials prepared by this dilute process are found to work very well in both aqueous dispersion and prototype dentifrice compositions as confirmed by hydraulic conductance tests and SEMs as shown in Figures 1 and 2 (two similarly prepared dentin surfaces, untreated and treated).

In the precipitation-based process of the present invention, sodium silicate [CAS# 1344-09-8] may be substituted as the silicon source. The addition of an appropriate amount of a soluble calcium source, i.e. Ca(NO₃)₂, to a solution of sodium silicate precipitates a calcium-containing silicate whose composition is similar to previously described materials. The amount of Ca⁺⁺ to be added to the silicate solution was determined by both stoichiometric calculations and experimental design.

The precipitation-based process of the present invention has several advantages over other processes. This process produces no by-products that may contaminate the final product after incorporation into a standard dentrifice, i.e., ethanol or residual TEOS. In addition, the chemistry of the reaction in this process is such that particle size can more easily be controlled during the reaction, thus eliminating the need for further size reduction and milling steps which are not only costly but also may increase the chance for contamination of the final product. Further, the reaction requires only two ingredients, sodium silicate and the appropriate calcium source. Both of these chemicals are readily available from common chemical supply companies and are relatively inexpensive compared with the other processes.

In this process, the material, once precipitated and separated from the remaining solvent, does not require any special processing, including washing, drying at high heat or supercritical drying; nor does the material involve a calcination procedure or organic solvent extraction.

It is additionally preferred, although not critical, that in the process, the precipitated material is chemically controlled to produce amorphous material of sufficiently small particle size range as to not require additional milling, grinding or size reduction. Thus, one avoids the possibility of sample contamination and additional processing costs.

The preferred process of chemical reactions performed under ambient conditions produces a highly porous, calcium-containing silica similar to bioactive glass prepared by the traditional sol-gel process in biological activity. This inorganic material does not resemble the heat-sintered bioactive glass material currently available in other ways.

It is most preferred that the reaction produces a calcium-containing oxide material based solely on a two component system, CaO-SiO₂; and that the reaction occurs at room temperature in solvents familiar to the health care field as well as safe and well-known to production facilities personnel.

The bioactive materials prepared in the examples that follow are suitable for oral care compositions for use in the treatment of dentin hypersensitivity.

**Examples** The following examples are provide for illustrative purposes only.

In examples tailored toward the treatment of dentin hypersensitivity, experiments were conducted using a modified *in vitro* model of dentin sensitivity described in U.S. Patent 5,270,031. In this method, intact human third molars free of caries or restorations are sectioned perpendicular to the long axis of the tooth with a metallurgical saw into sections approximately 1 mm. thick. Sections containing dentin and free of enamel are retained for testing. These sections are then etched with an EDTA (ethylenediamine tetraacetic acid) solution to remove the smear layer. The disk is mounted in a split chamber device as reported in Journal of Dental Research 57:187 (1978). This special leak proof chamber is connected to a pressurized fluid reservoir containing a tissue culture fluid intended to mimic the osmolarity of human body fluid. By using a mixture of pressurized N₂ and CO₂ gas, the fluid can be maintained at physiological pH. The apparatus includes a glass capillary tube mounted on a ruler or other measuring instrument. An air bubble is injected into the glass capillary tube and by measuring the displacement of the bubble as a function of time the fluid flow through the dentin disk can be measured. It has been reported that fluid actually flows out of dentin tubules from the interior of a normal tooth.

Following measurements of the baseline fluid flow in the dentin disk, the experimental mixture, dentifrice, gel or mouthwash is applied to the external disk surface in a manner that would mimic its use clinically. After a defined application period, the experimental material is rinsed off and post-application (Ipacute) hydraulic conductance is measured. In this fashion various experimental materials both alone and as components of final products can be tested for the ability to obstruct fluid flow in the dental tubules. The percent flow reduction induced by application of the experimental material can then be calculated.

For all examples given, saliva incubation was necessary to achieve flow reductions. For the purpose of studying the mineralizing potential of novel agents, an extended time period is necessary as well as a modification of the standard dentin hydraulic conductance measurement. After initial post-application measurements are taken, the split chamber is removed from the apparatus and, a reservoir of sterile filtered human saliva is attached to the chamber allowing the fluid to bathe the treated occlusal dentin surface. The exterior of the chamber is wrapped in parafilm to avoid contact with air and resulting fluid loss. The unit is then placed in an incubator kept at body temperature. After a sufficient time, the unit is removed, the saliva reservoir is discarded and the split chamber is re-attached to the hydraulic conductance apparatus. At this time, dentin fluid flow can be re-measured and the experimental material can be re-applied. In the examples that follow, the flow reductions reported were obtained after three treatments and 40 hours of saliva incubation.

In the examples that follow as throughout this specification and claims, all temperatures are in degrees centigrade and all parts and percentages are by weight unless otherwise indicated.

Example 1 (Precipitation-Based Process Producing Inorganic Particles). A precipitate was prepared using commercially available calcium nitrate and sodium silicate solution. To 72.8 wt. % of a 40% sodium silicate solution, 27.2 wt. % of a 75% calcium tetrahydrate solution in deionized water was added, with the mixer running at high speed to provide maximum agitation to the sodium silicate solution. Precipitation occurred immediately. The precipitate (Calcium Silicate, Inorganic Precipitate or "CSIP") was dried at 60°C for 40 hours.

In example 1, the CSIP particle size was reduced by milling (e.g., using an air impact mill, simple ball mill, etc.). However, it is possible to alter the particle size by altering the pH or diluting the sodium silicate solution while maintaining the high calcium nitrate concentration of lowering the final particle size of the precipitate. Superior tubule occluding efficacy was judged both by hydraulic conductance and SEMs as shown in Figures 1-2 (untreated and treated with the composition of Example 1).

Example 2 A toothpaste composition which further aids in the alleviation of pain when used on hypersensitive teeth was prepared using the following formulation. The weight percent given for each ingredient is based on a value of 100% for the total formulation.

| INGREDIENT | WEIGHT % |
|---|---|
| Water | 48.4 |
| Calcium Silicate (derived from a Sodium Silicate Solution [1344-09-8] and Ca(NO₃)₂ [13477-34-4]) | 5.0 |
| Sodium laurel sarcosinate | 0.4 |
| Sodium saccharin | 0.3 |
| Sodium fluoride | 0.2 |
| Hydroxyethylcellulose | 2.3 |
| Hydrated silica abrasive | 6.0 |
| Sodium lauryl sulfate | 1.2 |
| Glycerin | 23.0 |
| Hydrated silica thickener | 8.0 |
| Peppermint Oil | 0.2 |

Example 3. Formulations of a Medicinal-Type Mouthwash and Fluoride Oral Rinse are as follows in weight percent (adjusted to pH 6):

| INGREDIENT | Medicinal-Type | Fluoride Oral |
|---|---|---|
| Inorganic Precipitated Material | 1.0 | - |
| Ethyl Alcohol | 15.0 | - |
| Glycerin | 20.0 | 15.0 |
| Polyethylene glycol | 0.5 | . |
| Water | 61.5 | 73.5 |
| Caramel Color | to desired shade | - |
| Sodium Saccharin | 0.03 | 0.05 |
| Ormosil | - | 2.0 |
| Alcohol | - | 5.0 |
| Spearmint Oil | - | 0.25 |
| Poloxamer 338 | - | 1.0 |
| Sodium fluoride | - | 0.05 |
| Sodium benzoate | - | 0.1 |
| FD&C dyes | - | to desired color |

## Claims

1. The use of an amorphous bioactive particulate material consisting of calcium, silicon and oxygen, which material is a precipitated reaction product of a soluble calcium source and a silicate solution in the preparation of an oral care composition for treating dentin hypersensitivity.

2. The use according to claim 1 wherein said amorphous material is a precipitated reaction product of a soluble calcium source and a dilute silicate source selected from an aqueous potassium or sodium silicate solution.

3. The use according to claim 1 or 2, in which the said material has a particle size in the range of about 10 microns or less.

## Patentansprüche

1. Verwendung eines amorphen, bioaktiven, teilchenförmigen Materials, das aus Calcium, Silizium und Sauerstoff besteht, wobei das Material ein präzipitiertes Reaktionsprodukt einer löslichen Calciumquelle und einer Silicatlösung ist, bei der Herstellung einer Mundpflegezusammensetzung zur Behandlung einer Dentin-Überempfindlichkeit.

2. Verwendung gemäß Anspruch 1, worin das amorphe Material ein präzipitiertes Reaktionsprodukt einer löslichen Calciumquelle und einer verdünnten Silicatquelle ist, die ausgewählt ist aus einer wäßrigen Kalium- oder Natriumsilicatlösung.

3. Verwendung gemäß Anspruch 1 oder 2, worin das Material eine Teilchengröße im Bereich von circa 10 µm oder weniger hat.

## Revendications

1. Utilisation d'un matériau particulaire bioactif amorphe consistant en calcium, silicium et oxygène, lequel matériau est un produit de réaction précipité d'une source de calcium soluble et d'une solution de silicate dans la préparation d'une composition d'hygiène orale pour le traitement de l'hypersensibilité de la dentine.

2. Utilisation selon la revendication 1, dans laquelle ledit matériau amorphe est un produit de réaction précipité d'une source de calcium soluble et d'une source de silicate diluée choisie parmi une solution aqueuse de silicate de potassium ou de sodium.

3. Utilisation selon la revendication 1 ou 2, dans laquelle ledit matériau a une dimension particulaire comprise dans la gamme d'environ 10 µm ou moins.
